# EUROPEAN PATENT SPECIFICATION

(11) **EP 4 452 130 B1**
(45) Date of publication and mention of the grant of the patent: **07.05.2025**
(21) Application number: 22834909.8
(22) Date of filing: 08.12.2022
(51) Int. Cl.: A61C 17/22, A61C 17/16, A61C 9/00

(54) **PERSONAL CARE DEVICES, COMPUTER PROGRAM AND NON-THERAPEUTICAL METHOD OF PROCESSING CAPTURED IMAGES**
KÖRPERPFLEGEGERÄTE, COMPUTERPROGRAMM UND NICHT-THERAPEUTISCHE METHODE ZUR VERARBEITUNG AUFGENOMMENER BILDER
APPAREILS DE SOINS PERSONNELS, PROGRAMME INFORMATIQUE ET MÉTHODE NON THÉRAPEUTIQUE DE TRAITEMENT DES IMAGES CAPTURÉES

(30) Priority: 23.12.2021 EP 21217440
(43) Date of publication of application: 30.10.2024
(73) Proprietor: Koninklijke Philips N.V., 5656 AG Eindhoven (NL)
(72) Inventor: VAN DEN DUNGEN, Wilhelmus, Andreas, Marinus, Arnoldus, Maria, 5656 AG Eindhoven (NL); JOHNSON, Mark, Thomas, 5656 AG Eindhoven (NL); KOOIJMAN, Gerben, 5656AG Eindhoven (NL)
(74) Representative: Philips Intellectual Property & Standards
(86) International application number: PCT/EP2022/084893
(87) International publication number: WO 2023/117443

(56) References cited:
- US-A1- 2019 236 935
- US-A1- 2020 179 089

## Description

### FIELD OF THE INVENTION

The present invention relates to the field of personal care devices, and in particular to the field of personal care devices that capture digital impressions of one or more features of a user.

### BACKGROUND OF THE INVENTION

It is known to provide personal care devices with the ability or functionality to capture digital impressions (e.g. images) of a feature or part of a user during use. For example, Intra-Oral Scanner (IOS) devices use projected light (e.g. laser or structured light) and an image sensor to capture images of the dentogingival tissues. These images can be processed to create a three-dimensional (3D) model of the scanned surface. Data provided by IOS can therefore be useful for oral care, including the detection of common dental pathologies such as caries, tooth discoloration, misalignment. Also, it may be advantageous to capture and compare repeated IOS images, to enable identification of changes in dentogingival tissues over time for example.

Because personal care devices, such as electric brushing or shaving devices, are used on a regular (e.g. daily) basis, they may provide a suitable vehicle to regularly capture images of a part of a user. Accordingly, there is trend to integrate cameras or imaging sensors into personal care devices, such as toothbrushes for example. However, because a main usage characteristic of such a personal care device may be its vibration or cyclical/periodic movement, images acquired by an image capture device integrated with a personal care device are typically distorted and/or blurred by the movement/vibration of the device.

Although image stabilization techniques are known for portable handheld devices (e.g. smart phone, digital photo cameras, etc.), such techniques are only designed and optimized for low frequency (e.g. <10 Hz), erratic (e.g. non-periodic, random, etc.) user-induced motions. Because a personal care device (such as a toothbrush) typically moves or vibrates at a much higher periodic frequency (e.g. >20-300 Hz), there remains a need to stabilize the image capture process and/or make the image acquisition process robust to device movements/vibrations generated by a personal care device.

US2019236935A1 discloses an electrical hand-held device for body treatment, having a treatment part for performing at least one electrically operated body treatment function, and having a handle body for holding the hand-held device. which further includes an electronic component for controlling the at least one electrically operated body treatment function.

### SUMMARY OF THE INVENTION

The invention is defined by the claims.

According to examples in accordance with an aspect of the invention, there is provided a personal care device comprising:
an image capture device adapted, in use, to capture images of one or more features of a user; and
vibratory means adapted to vibrate the personal care device so that, in use, the personal care device (and the image capture device) vibrates with a vibration cycle having a vibration frequency; and
a sensor arrangement adapted to sense an operating parameter of the personal care device; and
a control unit adapted to determine a target part of the vibration cycle based on the sensed operating parameter and to control the image capture device based on the target part of the vibration cycle.

Proposed concepts thus aim to provide schemes, solutions, concepts, designs, methods and systems pertaining to aiding and/or improving image acquisition from a vibratory personal care device having an image capture device the image of which is influenced by the device vibration. This may either be a camera fixed rigidly to the vibrating device which may then vibrate with the device. Alternatively the camera sensor itself may be situated in a stationary part of a device (e.g. a brush handle) whilst the optical system of the device (like an optical fiber, the imaging lens of the optical system) are subject to the device vibrations. In particular, embodiments of the invention propose identifying a target part (e.g. low velocity/motion part) of the vibration cycle based on a sensed operating parameter of the personal care device, and then controlling the image capture device based on the identified target part of the vibration cycle. Through control of the image capture device according to a target part of the vibration cycle, improved images (e.g. images exhibiting less blur and/or distortion) may be obtained. **In** this way, improved images may be obtained from a vibratory personal care device.

In particular, it is proposed that, to reduce or minimize distortions in images acquired from vibrating personal care device (in use), timing and/or settings of the image capture device may be adapted based on the vibration cycle. For instance, a timing of image data capture by the image capture device may be synchronised with the target part of the vibration cycle, thus ensuring the image data capture only occurs during a preferred or optimal part of the vibration cycle.

It is also proposed to determine the target part of the vibration cycle by sensing operation of the personal care device. By way of example, a movement and/or a drive signal of the vibratory means may be monitored and then analysed to determine a period/portion of the vibration cycle which exhibits low/zero motion of the personal care device.

In other words, embodiments propose to adapt image capture based on one or more sensed operating parameters of the personal care device, so as to provide images of improved quality and/or enable the use of simpler/cheaper image capturing devices. Embodiments may therefore facilitate stable and high-quality image acquisition during tooth brushing. Such embodiments may be particulalry relevant to teledentistry propositions, for example, by enabling improved imaging of a user's tooth, gum, tongue, etc. For instance, images obtained by proposed embodiments may aid dental care treatments and/or decision making. Accordingly, embodiments may be used in relation to dental treatment selection so as to support a dental care professional when selecting treatment for a subject.

Further, embodiments may facilitate image-based diagnostics, superior location sensing, therapy planning (e.g. orthodontics, aligners) and/or dental treatment monitoring.

By being integrated into the normal brushing regimen of a user (instead of using separate devices such as smart phones, dental endo-scopes or handheld intraoral scanners, embodiments may support improved dental care. Improved image-based dental care may therefore be provided by proposed concepts.

It has been realized that by controlling image capture device based on target part of the vibration cycle of the personal care device, higher quality images (in terms of reduced blur and/or distortion) may be obtained, thus aiding oral/dental care decision making.

Embodiments may therefore provide the advantage that decision making in selecting an oral care treatment can be improved through the use of images captured by a virbatory personal care device. For example, embodiments may enable a larger number of oral care treatment options to be available (e.g through the provision of more and/or improved information about a subjectt's oral health).

The image capture device may comprise an illumination device configured, in use, to illuminate a part of the user's oral cavity. The control unit may then also be adapted to control the image capture device to synchronise a timing of illumination with the target part of the vibration cycle. This will help to ensure adequate lighting conditions for reducing image blur and/or distortion whilst ensuring sufficient total illumination. It may also help to reduce power consumption by restricting illumination to only occur at certain times. That is, activation of the illumination device for unnecessary time periods where for example during the high speed motion and thus harder to capture bright and clear images in that period may be avoided.

In some embodiments, the image capture device may comprise a camera, and the control unit may be adapted to synchronise signal acquisition from the camera with the target part of the vibration cycle. Embodiments may thus be employed in conjunction with different types of image capture devices, including, for example, global shutter cameras or free-running rolling shutter cameras that are out of synchronisation with vibration of the personal care device (and the image capture device). For example, by timing exposure or data acquisition of/from the camera based on the target part of the vibration cycle, image data that is sharp and clear may be acquired. This image data may be combined with image data from different portions of different frames of the camera, thus facilitating construction of a single high quality image (from a plurality of captured frames).

In an exemplary embodiment, the control unit may be adapted to control the image capture device to synchronise a timing of image capture with the determined target part of the vibration cycle. For instance, the start/beginning of an exposure window (i.e. the timing of the start of the exposure period) may be aligned with the timing of a low angular velocity of the personal care device during its vibration cycle. Such a concept of synchronizing image capture with a low velocity period/window in the vibration cycle of the personal care device may further reduce blur and/or distortion in captured images.

In some embodiments, the control unit may be configured to generate a timestamp based on the target part of the vibration cycle, the timestamp being configured to identify a timing of the target part of the vibration cycle. Embodiments may thus provide a concept for identifying the timing of the target part of the vibrations, thereby assisting synchronisation with the target part of the vibration cycle. Such a concept is particularly useful in combination with free running image capture devices.

In some embodiments, the control unit may also be configured to control an exposure parameter of the image capture device based on the target part of the vibration cycle. For instance, the exposure parameter may comprise an exposure duration of the image capture device, and the control unit may then be adapted to set the image capture exposure duration. In this way, exposure of the image capture device may be set to be fast enough relative to the camera vibration to reduce or minimise image distortion or bur. That is, the image capture exposure time may be set to be less than or equivalent to the duration/period of minimal motion of the personal care device.

In an embodiment, the vibratory means may comprise a vibrator, and the sensor arrangement may comprise a drive sensor adapted to sense a drive signal of the vibrator. The control unit may then be adapted to determine the target part of the vibration cycle based on a time period when then sensed drive signal meets a predetermined drive signal requirement. For instance, the sensed drive signal may be representative of a drive current or voltage of the vibrator, and the predetermined drive signal requirement may be that the magnitude of the drive current or voltage cross a threshold value. For example the drive current may exceed a threshold, the drive voltage may fall below a threshold, etc. In this way, sensing of the drive signal may be employed to determine the target part of the vibration cycle (e.g. identify the timing of a low velocity period within the vibration cycle). Simple voltage or current monitoring techniques may therefore be employed to accurately identify the timing(s) of low velocity of the personal care device.

In some embodiments, the sensor arrangement may comprise a movement sensor adapted to sense an angular velocity of a part of the personal care device. The control unit may then be adapted to determine the target part of the vibration cycle based on a time period when the sensed angular velocity meets a predetermined velocity requirement. For instance, the predetermined velocity requirement may be that the magnitude of the sensed angular velocity does not exceed a threshold value. Thus, movement/motion sensing concepts may be employed to determine the target part of the vibration cycle (e.g. identify the timing of a low velocity period within the vibration cycle). Simple movement sensing techniques and/or apparatus may therefore be employed to accurately identify the timing(s) of low velocity of the personal care device.

In an embodiment, the sensor arrangement may comprise a hall sensor adapted to sense a magnetic field at a part of the personal care device. The control unit may then be adapted to determine the target part of the vibration cycle based on a time period when then sensed magnetic field meets a predetermined magnetic field requirement. Sensing of the magnetic field may thus be employed to determine the target part of the vibration cycle (e.g. identify the timing of a low velocity period within the vibration cycle). Existing hall sensors that are already integrated within conventional personal care devices may therefore be leveraged to provide new and/or extended functionality, for example.

In other embodiments, the sensor arrangement comprises a vibration sensor adapted to sense an acceleration or excursion of a vibration of a part of the personal care device. The control unit may then be adapted to determine the target part of the vibration cycle based on a time period when the magnitude of the sensed vibration meets a predetermined acceleration or excursion requirement. For instance, the predetermined acceleration or excursion requirement may be that the acceleration of the sensed vibration exceeds a vibration threshold value. Similarly the low speed is usually associated with the extremities of the motion, whereby the predetermined acceleration or excursion requirement may be that the excursion of the sensed vibration exceeds a vibration threshold value. Thus, vibration sensing concepts/apparatus may be employed to determine the target part of the vibration cycle (e.g. identify the timing of a low velocity period within the vibration cycle). Existing and/or simple vibration sensing techniques and/or apparatus may therefore be employed to accurately identify the timing(s) of low velocity of the personal care device.

Whilst the above is also relevant for a vibration sensor adapted to sense an acceleration or excursion positioned on the brush head it is clearly possible that the sensor is positioned in a brush handle. This may result in a more complex motion of the vibration sensor. However in this case also determining the low velocity periods will be possible using other predetermined requirements of the acceleration and/or excursion, which whilst complex will in general remain periodic in nature.

The personal care device may comprise a toothbrush, and may preferably comprise an oral care device (such as an electric toothbrush) that is adapted to vibrate in use. In other embodiments, the personal care device may comprise a mouthpiece, shaver, or skin cleansing device that is adapted to vibrate in use. One or more proposed concept(s) may therefore be employed in a range of different personal care devices. Embodiments may therefore have wide application in the field of personal care devices (and image capture and/or processing concepts for images captured by vibratory personal care devices).

According to another aspect of the invention, there is provided a method of controlling a personal care device, wherein the personal care device comprises: an image capture device adapted, in use, to capture images of one or more features of a user; vibratory means adapted to vibrate the personal care device so that, in use, the personal care device vibrates with a vibration cycle having a vibration frequency; and a sensor arrangement adapted to sense an operating parameter of the personal care device, and wherein the method comprises: determining a target part of the vibration cycle based on the sensed operating parameter; and controlling the image capture device based on the determined target part of the vibration cycle.

According to yet another aspect of the invention, there is provided a computer program comprising computer program code means which is adapted, when said computer program is run on a computer, to implement a method according to proposed embodiment.

Thus, there may also be provided a computer system comprising: a computer program product according to proposed embodiment; and one or more processors adapted to perform a method according to a proposed concept by execution of the computer-readable program code of said computer program product.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 is a simplified schematic block diagram of an electric toothbrush according to a proposed embodiment;
Figure 2A is a graph showing an exemplary variation in angle θ (of displacement) and angular velocity of the brush head of Figure 1 over time as the brush head vibrates, wherein the variation in displacement angle θ of the brush head is depicted by the solid line, and wherein the variation in angular velocity v of the brush head is depicted by the dashed line;
Figure 2B depicts graphs showing the corresponding variations in motor signals and motor current for the variations of brush head angle and angular velocity shown in Figure 2A;
Figure 3 is a simplified schematic block diagram of a mouthpiece according to a proposed embodiment;
Figures 4A-4D illustrate a proposed concept of synchronising data capture with a target part of the vibration cycle and the reconstructing a single, higher quality image from the captured data, wherein the concept is employed in conjunction with a free rolling camera and synchronised flash (i.e. pulses of illumination synchronized with the vibration cycle);
Figures 5A-5D illustrate a proposed concept of synchronising data capture with a target part of the vibration cycle and the reconstructing a single, higher quality image from the captured data, wherein the concept is employed in conjunction with a free rolling camera and continuous illumination; and
Figure 6 is a simplified block diagram of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The mere fact that certain measures are recited in mutually different dependent claims does not indicate that a combination of these measures cannot be used to advantage.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

It should be understood that the Figures are merely schematic and are not drawn to scale. It should also be understood that the same reference numerals are used throughout the Figures to indicate the same or similar parts.

The invention proposes concepts for aiding and/or improving image acquisition from a vibratory personal care device having an image capture device. In particular, embodiments may provide a system, device and/or method which identifies a target part of the vibration cycle based on an operation of the personal care device. Control of the image capture device is then undertaken according to the identified target part of the vibration cycle. Through such control of the image capture device, improved images (e.g. images exhibiting less blur and/or distortion) may be obtained.

In particular, it is proposed that, to reduce or minimize distortions in images acquired from a vibrating personal care device, the image capture device may be adapted according to the vibration cycle of the vibrating personal care device (in use). More specifically, it has been realised that one or more parts of the vibration cycle may be better suited to image capture, for example due to exhibiting reduced motion/movement. It has further been realised that the timing(s) of such a part of the vibration cycle preferred (or optimal) for image capture may be determined based on operating parameters of the personal care device. Examples of such operating parameters include: vibrator/motor current or voltage; magnetic field; device motion; and angular velocity of a part of the personal care device. Simple detection of an operation of the personal care device to determine a preferred, optimal or target timing of image capture may therefore be employed, thus enabling improved or optimised image capture.

In other words, embodiments propose to adapt image acquistion to the vibration of the personal care device, so as to provide images of improved quality.

Embodiments may therefore facilitate stable and high-quality image acquisition during tooth brushing. Such embodiments may be particulary relevant to teledentistry propositions, for example, by enabling improved imaging of a user's tooth, gum, tongue, etc. For instance, images obtained by proposed embodiments may aid dental care treatments and/or decision making. Accordingly, embodiments may be used in relation to dental treatment selection so as support a dental care professional when selecting treatment for a subject.

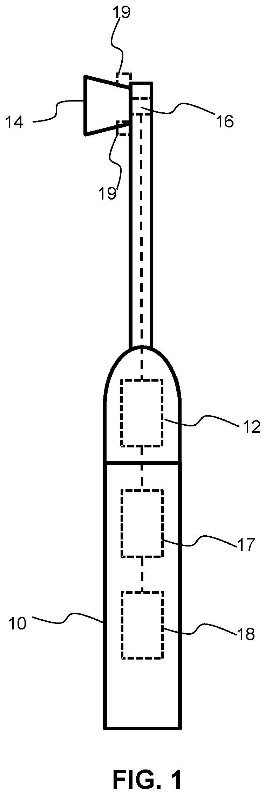

Referring to Figure 1, there is shown a simplified schematic block diagram of an electric toothbrush 10 according to a proposed embodiment. The electric toothbrush 10 comprises vibratory means 12 (specifically, a motor) that is adapted, in use, to vibrate a brush head 14 of the electric toothbrush 10 (with a frequency of about 200Hz).

The electric toothbrush 10 also comprises an image capture device 16 (e.g. digital camera) that is adapted, in use, to capture images of one or more oral features of a user. The electric toothbrush 10 is also provided with a sensor arrangement 17 that is adapted to sense an operating parameter of the personal care device, and a control unit 18 that is adapted to control the image capture device based on the target part of the vibration cycle.

More specifically, the motor 12 is configured to cause the brush head 14 to repeatedly rotate clockwise then anti-clockwise by around 10°-25° from a rest position in a periodic manner. In this way, the brush head 14 vibrates with a periodic vibration waveform.

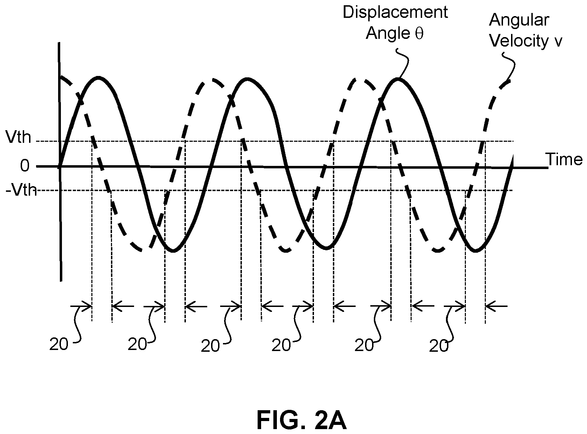

By way of further illustration, Figure 2A depicts a graph showing an exemplary variation in angle θ (of displacement) and angular velocity of the brush head 14 over time as the brush head 14 vibrates. The variation in displacement angle θ of the brush head 14 is depicted by the solid line, whereas the variation in angular velocity v of the brush head 14 is depicted by the dashed line.

It is seen from the variations depicted in Figure 2A that 'low speed' periods 20, i.e. time periods wherein the magnitude of angular velocity v of the brush head 14 is below a threshold value Vth, can be identified. More specifically, it is seen that, when the magnitude of the displacement angle θ reaches a maximum value, such that the gradient of displacement angle θ variation changes polarity (i.e. when the solid line changes direction), the angular velocity v of the brush head 14 is near-zero. It is proposed that these low speed periods 20 provide the most appropriate opportunities for image capture using an image capture device that may have limited speed and exposure tuning capabilities. Accordingly, embodiments are based on the concept(s) that timing and/or settings of the image capture device may be adapted according to the vibration cycle of the brush 14. For instance, one or more exposure settings of the image capture device 16 may be controlled based on the vibration cycle of the brush 14. That is, embodiments propose to adapt image acquistion to the vibration of the personal care device, so as to provide images of improved quality.

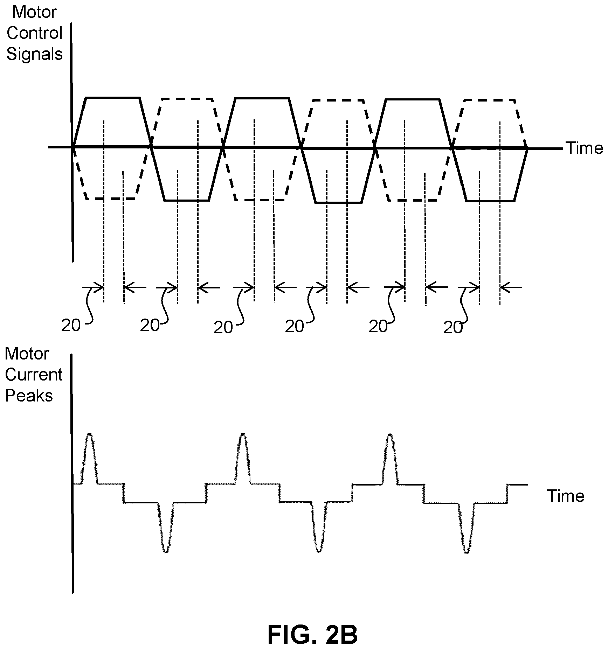

Figure 2B depicts graphs showing the variations in motor signals and motor current for the variations of brush head 14 shown in Figure 2A. The motor control signal is a (substantially) square wave signal. It is seen that the motor signal is "on" during the low speed periods 20 (e.g. when reversing the rotation angle movement direction of the brush head). From the bottom graph of Figure 2B, it is seen that the current is also related to the angular velocity: a high current peak represents the reversing of the rotation angle movement direction and getting it up to speed again; and a following (substantially) flat phase represents where the speed is kept constant;

Often in electronic drive arrangements, a peak in drive current is associated with a small dip in the drive voltage. For this reason also the drive voltage may be advantageously used to identify low speed periods.

In this example it is realised that the low speed period is related to the motor "on" signal, and the period of no speed is related to the peak current.

Accordingly, it is proposed that an operating parameter of the personal care device can be sensed/monitored to determine a timing of preferred, optimal or target period of image capture, such as the timing of the low speed periods 20 of the vibration cycle. As shown in Figure 2A, the operating parameter may comprise the motor 12 current (or signal) and, for this reason, the sensor arrangement 17 of the embodiment of Figure 1 comprises a drive sensor adapted to sense a drive signal of the vibrator, wherein the sensed drive signal is representative of a drive current or voltage of the vibrator.

Referring back to the embodiment of Figure 1, the control unit 18 is adapted to determine the target part of the vibration cycle based on a time period when the sensed drive signal meets a predetermined requirement. Specifically, the predetermined requirement is that the magnitude of the drive current or voltage cross a threshold value. For example the drive current may exceed a threshold, the drive voltage may fall below a threshold etc. In this way, the target part of the vibration cycle is design to coincide with a low speed period 20 of the vibration cycle.

The control unit 18 is then adapted to control the image capture device 16 based on the target part of the vibration cycle. Specifically, in this example, the control unit is adapted to control the image capture device 16 to synchronise a timing of image capture with the target part (i.e. low speed period 20) of the vibration cycle. The image capture device 16 is thus controlled so that image capture is synchronised with the low speed periods 20, thereby reducing an amount of movement that may cause blurring and/or distortion in a captured image.

To aid reduction in the exposure duration that is required to capture an adequately exposed image, the image capture device 16 of the embodiment of Figure 1 comprises illumination devices 19 (e.g. LEDs, lasers) that are configured, in use, to illuminate a part of the user's oral cavity. That is, where the sensitivity (e.g. ISO) and/or the lens aperture of the image capture device 16 may be fixed and/or limited, a reduction in the required length of exposure to capture an adequate amount of light may be obtained through illumination of the user's oral cavity by the illumination devices 19. Further, the control unit 18 of this exemplary embodiment is adapted to control the image capture device 16 to synchronise a timing of illumination by the illumination devices 19 with the vibration cycle. For instance, the illumination by the illumination devices 19 may be synchronised with the low speed periods 20. This may help to reduce or minimise power consumption whilst still ensuring suitable illumination to facilitate a reduced/shortened exposure duration of the image capture device 16 during the low speed periods. In other embodiments, however, the illumination devices 19 may be controlled to provide continuous illumination, e.g. illumination may be provided by the illumination devices 19 for the duration of the vibration cycle.

Although the embodiment of Figure 1 has been described as employing a brush head 14 that repeatedly rotates clockwise then anti-clockwise by around 10°-25° from a rest position in a periodic manner, it will be appreciated that other embodiments may employ a brush head that vibrates in a different manner. For instance, alternative embodiments may comprise a brush head that rotates or that shakes (left and right, or up and down) repeatedly, i.e. repeatedly displaces (laterally or vertically) in opposite directions from a rest position. Such embodiments will still exhibit a cyclical vibration pattern having variations in displacement and velocity over time that form periodic waveforms as depicted in Figure 2, and thus have repeating 'low speed' periods that are identifiable (e.g. according to the drive parameters and/or control of the vibratory means).

That is, although the proposed concept(s) have been described above with reference to rotating / angular motion of the vibrating part of the personal care device, the proposed concept(s) may be employed in other vibratory personal care devices exhibiting repetitive vibratory motion. Also, in case of multi-dimensional motion, periods of low/zero speed may be identified with respect to multiple dimensions and used to control one or more exposure parameters of the image capture device.

Also, although the embodiment of Figure 1 employs a concept of identifying periods of low or no angular velocity by monitoring the motor drive signal (e.g. drive current or voltage), other parameters of the personal care device may be monitored to identify the vibration cycle (and thus infer the target part of the vibration cycle). Examples of other parameters that may be monitored include: magnetic field; device motion; and angular velocity of a part of the personal care device. Simple detection of an operation of the personal care device to determine a preferred, optimal or target timing of image capture may therefore be employed, thus enabling improved or optimised image capture.

Simply by way of example, in a modified version of the embodiment of Figure 1, the sensor arrangement comprises a movement sensor that is adapted to sense an angular velocity of a part of the personal care device. Such a sensor can be a gyroscope, compass or other angular sensing device. The sensor arrangement may also comprise an accelerometer/vibration sensor positioned in the handle of the personal care device for determining the frequency of motion and phase. The control unit is then adapted to determine the target part of the vibration cycle based on a time period when then sensed angular velocity meets a predetermined velocity requirement. For instance, the predetermined velocity requirement may be that the magnitude of the sensed angular velocity does not exceed a velocity threshold value, thus identifying the low speed periods 20 of the vibration cycle by sensing an angular velocity of a part of the personal care device.

In another example, the sensor arrangement may comprise a hall sensor that is adapted to sense a magnetic field at a part of the personal care device. The control unit may then be adapted to determine the target part of the vibration cycle based on a time period when then sensed magnetic field meets a predetermined magnetic field requirement. Thus, an existing hall sensor that is already integrated within a conventional personal care device may be leveraged to provide new, improved and/or extended functionality.

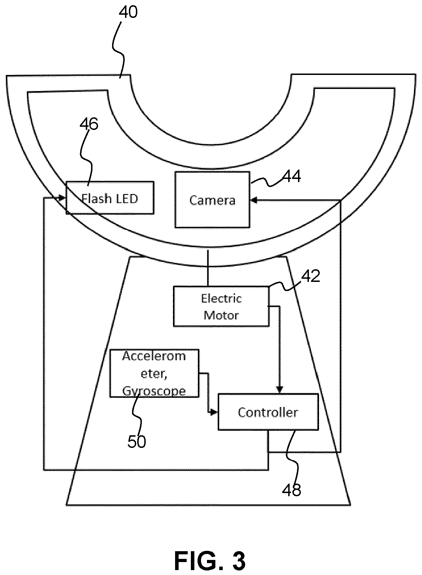

Referring to Figure 3, there is shown a simplified schematic block diagram of a mouthpiece 40 according to another embodiment. The mouthpiece 40 is adapted to be inserted into a user's mouth and vibrate (in use) for oral cleaning purposes.

The mouthpiece 40 comprises vibratory means 42 (specifically, an electric motor) that is adapted, in use, to cause the mouthpiece to vibrate with periodic vibration waveform (having a frequency in the range of 100Hz-500Hz).

The mouthpiece 40 also comprises an image capture device 44 positioned in the tray of the mouthpiece and adapted, in use, to capture images of one or more oral features of the user. A flash LED 46 is also provided in the tray of the mouthpiece for illuminating the oral cavity of the user, in use. The image capture device 44 and LED 46 are configured to be controlled by a control unit (i.e. controller) 48 of the mouthpiece.

In particular, the control unit 48 is configured to control an exposure parameter of the camera 44 and activation the flash LED 46 based on a target part of the vibration cycle of the mouthpiece 40. For determining a target part of the vibration cycle of the mouthpiece 40, the mouthpiece 40 comprises a sensor arrangement 50 that is adapted to sense an operating parameter of the oral care. Specifically, in this example, the sensor arrangement 50 comprises an accelerometer and gyroscope for detecting variations in displacement and velocity of the mouthpiece 40. Information about the detected variations in displacement and velocity of the mouthpiece 40 is provided to the control unit 48 and, based on this information, the control unit 48 determines a target part of the vibration cycle of the mouthpiece 40.

As already explained above with reference to the embodiment of Figure 1, the control unit 48 determines a target part of the vibration cycle (e.g. a period of 'low speed/velocity' of the mouthpiece 40, i.e. time period wherein the magnitude of velocity of the mouthpiece 40 is below a predetermined threshold value). The control unit 48 then controls the camera so that image capture is timed to occur during the target part of the vibration cycle (e.g. during identified periods of 'low speed/velocity' of the mouthpiece 40).

However, it is noted that, in this embodiment, the image capture device 44 comprises a rolling shutter camera configured to operate at an image capture frame rate. With respect to such a rolling shutter camera, trade-offs in operating characteristics may be required. For example, rolling shutter cameras may be more cost effective (e.g. cheaper) but may take longer for a full image capture depending on the acquisition settings and/or capabilities. An 'exposure period" is then considered to be the time period for which the rolling shutter camera is acquiring image data. For instance, image data may be acquired by the rolling shutter camera 44 for half of a frame, thus resulting in the effective exposure duration being half the period of the rolling shutter camera frame. Similarly the image data may be acquired by the rolling shutter camera 44 for one tenth of a frame, thus resulting in the effective exposure duration being one tenth of the period of the rolling shutter camera frame. Accordingly, the control unit 48 is thus further adapted to control an exposure parameter taking account of the frame rate of the rolling shutter camera.

In this example, a first exposure parameter comprises a start of exposure timing (i.e. the time at which image data being capture by the rolling shutter camera is commenced/started), and the control unit 48 is adapted to control the start of capture of image data by the rolling shutter to be synchronised with the vibration cycle. Specifically, the control unit 48 is adapted to synchronise the start of image data capture from the rolling shutter camera with the low speed periods of the vibration cycle (identified based on information from the sensor arrangement 50). In this way, the image capture device 44 is controlled so that image data capture is synchronised with the low speed periods, thus reducing an amount of movement that may cause blurring and/or distortion in a captured image.

Furthermore, in this example, a second exposure parameter comprises an exposure duration of the image capture device 44 (i.e. length of image data capture from the rolling shutter camera), and the control unit 48 is adapted to set the image data capture duration to be less than or equal to a target exposure duration value. By way of example, the target exposure duration value may be determined based on a parameter of the periodic vibration waveform and a resolution parameter of the camera. For instance, the at least one resolution parameter may comprise one or more of: sensor pixel width; sensor pixel length; sensor spacing; and sensor density. In this way, the exposure duration may be set to an optimum value relation to the imaging sensor exposure shift during specific moments of motion, so as to prevent image exposure across multiple sensor elements/pixels. That is, an exposure duration of the image capture device may be set to be fast enough relative to the camera vibration to reduce or minimize image distortion or blur. This may, for example, result in a total image capture exposure duration being less than or equal to an eighth of the period of the vibration waveform. Even more preferably, the image capture exposure duration may be less than or equal to a tenth of the period of the vibration waveform of the mouthpiece 40.

Moreover, for the case of a rolling shutter camera for example, it may be even more preferable to limit the exposure duration further on a line-by-line basis to be equivalent to less than one pixel shift on the image capture device - that is, the shift of the image during motion that is within the pixel resolution. In this way, the total exposure duration may be shared (i.e. divided) across all rows of the camera sensor, so that light is incident on the sensor for short pulsed exposure period per row, with the cumulative total duration of all of the pulses adding up to the total exposure duration. This may be implemented by exposing each row of the sensor to incident light for a fraction of the exposure duration or by pulsing an illumination source for fraction of the exposure duration with the pulsing synchronized with the row exposure time of each row of the rolling shutter camera sensor. Here, reference to pixel may encompass a single sensing element of an image sensor or a sensing element (single sensor) of an image sensor array. That is, a pixel may be thought of as being a single element of a sensed image, wherein the sensed image is formed from a plurality of sensed elements (i.e. pixels).

As an example, with the distance to the object fixed, and motion fixed, the pixel shift speed may be calculated. For instance, if the image capture device has a sensor with 1024*768 pixels, the image area captured being 10 mm wide, and motion image shift being 2 mm left and 2 mm right with a vibration period of 2 ms. Movement will thus be 4mm in ½ period (1ms), and 4mm is covered by 4/10* 1024 = 410 pixels. A single pixel image movement takes 1ms/410 = 2.4µs. Allowing for ½ a pixel shift during exposure would result in an exposure time of 1.2µS. Thus, a total exposure time is the number of rows in the image times the line exposure time i.e.768 x 1.2µs = 0.92msec. In such a manner, the short pulsed exposure periods are distributed evenly across the entire frame period and exposure is optimized for and during the low speed motion periods.

In other cases like high framerates and special camera's where per line or per pixel the exposure can be adjusted, an optimum per pixel, line or frame could be set during the image acquisition and in relation to the ideal moments of capture where for example the speed of motion is low.

That is, for image acquisition using a rolling shutter camera 44, it is proposed to adapt the exposure duration to be fast relative to the camera vibration, e.g. effective exposure time (i.e. image data acquisition time by the rolling shutter camera 44) is adapted to be, at most, equivalent to the duration/period of minimal motion of the mouthpiece 40.

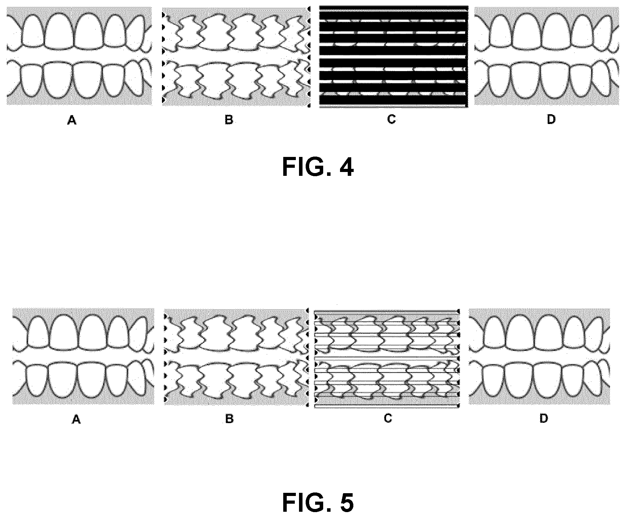

Referring to Figures 4 and 5, illustrations are provided to demonstrate a proposed concept of synchronising data capture with a target part of the vibration cycle and the reconstructing a single, higher quality image from the captured data. In particular, Figures 4A-4D illustrate the concept employed in conjunction with a free rolling camera and synchronised flash (i.e. pulses of illumination synchronized with the target part of the vibration cycle).

Figure 4A illustrates the object being imaged, specifically teeth of a user.

Figure 4B shows a captured image frame with the personal care device vibrating at 200Hz and no compensation (i.e. not employing the proposed concept(s) of this invention).

Figure 4C shows a captured image with the personal care device vibrating at 200Hz and a synchronised flash (i.e. pulses of illumination synchronized with the target part of the vibration cycle of the personal care device). From Figure 4C it can be seen that lines of image data are acquired, wherein the captured lines comprise sharp, blue free data captured during low speed periods of the vibration cycle.

Figure 4D shows a reconstruction image that is reconstructed from the captured lines of image data (from different captured image frames).A full image may then be reconstructed from image data acquired from multiple different frames of the rolling shutter camera.

Conversely, Figures 5A-5D illustrate the concept employed in conjunction with a free rolling camera and continuous illumination.

Figure 5A illustrates the object being imaged, specifically teeth of a user.

Figure 5B shows a captured image frame with the personal care device vibrating at 200Hz and no compensation (i.e. not employing the proposed concept(s) of this invention).

Figure 5C shows a captured image with the personal care device vibrating at 200Hz and continuous illumination, wherein lines of image data are identified as being captured during the target part of the vibration cycle of the personal care device. These indicated lines comprise sharp, blue free data captured during low speed periods of the vibration cycle.

Figure 5D shows a reconstruction image that is reconstructed from the selected lines of image data (from different captured image frames).

It will be appreciated that the rolling shutter camera can be free running and out of synchronization with vibration of the personal care device (and thus the image data capture frequency). A full image may then be reconstructed from image data acquired from multiple different frames of the rolling shutter camera.

From the above description of various concepts and embodiments, it will be appreciated that there is proposed a method of controlling a personal care device, wherein the personal care device comprises: vibratory means adapted to vibrate the personal care device so that, in use, the personal care device vibrates with a vibration cycle having a vibration frequency; an image capture device adapted, in use, to capture images of one or more oral features of a user; and a sensor arrangement adapted to sense an operating parameter of the personal care device. The method comprises: determining a target part of the vibration cycle based on the sensed operating parameter; and controlling the image capture device based on the determined target part of the vibration cycle. Such a method may be employed in a processing system or computer, and such a system/computer may be integrated with a vibratory personal care device.

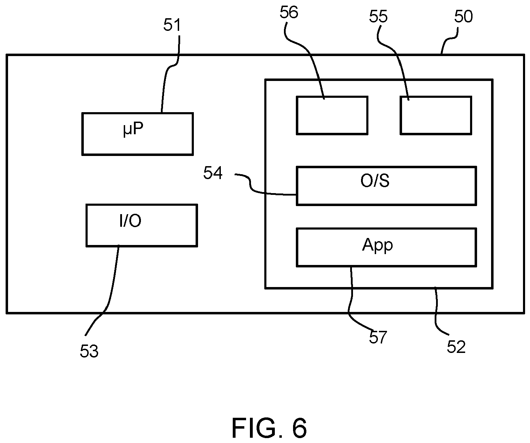

Figure 6 illustrates an example of a computer 50 within which one or more parts of an embodiment may be employed. Various operations discussed above may utilize the capabilities of the computer 50. For example, one or more parts of a system for providing a subject-specific user interface may be incorporated in any element, module, application, and/or component discussed herein. **In** this regard, it is to be understood that system functional blocks can run on a single computer or may be distributed over several computers and locations (e.g. connected via internet), such as a cloud-based computing infrastructure.

The computer 50 includes, but is not limited to, PCs, workstations, laptops, PDAs, palm devices, servers, storages, and the like. Generally, in terms of hardware architecture, the computer 50 may include one or more processors 51, memory 52, and one or more I/O devices 53 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 51 is a hardware device for executing software that can be stored in the memory 52. The processor 51 can be virtually any custom made or commercially available processor, a central processing unit (CPU), a digital signal processor (DSP), or an auxiliary processor among several processors associated with the computer 50, and the processor 51 may be a semiconductor based microprocessor (in the form of a microchip) or a microprocessor.

The memory 52 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 52 may incorporate electronic, magnetic, optical, and/or other types of storage media. Note that the memory 52 can have a distributed architecture, where various components are situated remote from one another, but can be accessed by the processor 51.

The software in the memory 52 may include one or more separate programs, each of which comprises an ordered listing of executable instructions for implementing logical functions. The software in the memory 52 includes a suitable operating system (O/S) 54, compiler 56, source code 55, and one or more applications 57 in accordance with exemplary embodiments. As illustrated, the application 57 comprises numerous functional components for implementing the features and operations of the exemplary embodiments. The application 57 of the computer 50 may represent various applications, computational units, logic, functional units, processes, operations, virtual entities, and/or modules in accordance with exemplary embodiments, but the application 57 is not meant to be a limitation.

The operating system 54 controls the execution of other computer programs, and provides scheduling, input-output control, file and data management, memory management, and communication control and related services. It is contemplated by the inventors that the application 57 for implementing exemplary embodiments may be applicable on all commercially available operating systems.

Application 57 may be a source program, executable program (object code), script, or any other entity comprising a set of instructions to be performed. When a source program, then the program is usually translated via a compiler (such as the compiler 56), assembler, interpreter, or the like, which may or may not be included within the memory 52, so as to operate properly in connection with the O/S 54. Furthermore, the application 57 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 53 may include input devices such as, for example but not limited to, a mouse, keyboard, scanner, microphone, camera, etc. Furthermore, the I/O devices 53 may also include output devices, for example but not limited to a printer, display, etc. Finally, the I/O devices 53 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 53 also include components for communicating over various networks, such as the Internet or intranet.

If the computer 50 is a PC, workstation, intelligent device or the like, the software in the memory 52 may further include a basic input output system (BIOS) (omitted for simplicity). The BIOS is a set of essential software routines that initialize and test hardware at startup, start the O/S 54, and support the transfer of data among the hardware devices. The BIOS is stored in some type of read-only-memory, such as ROM, PROM, EPROM, EEPROM or the like, so that the BIOS can be executed when the computer 800 is activated.

When the computer 50 is in operation, the processor 51 is configured to execute software stored within the memory 52, to communicate data to and from the memory 52, and to generally control operations of the computer 50 pursuant to the software. The application 57 and the O/S 54 are read, in whole or in part, by the processor 51, perhaps buffered within the processor 51, and then executed.

When the application 57 is implemented in software it should be noted that the application 57 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 57 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The proposed image capture and/or processing methods, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Figs. 1 and 3 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

## Claims

1. A personal care device comprising:
an image capture device (16) adapted, in use, to capture images of one or more features of a user;
vibratory means (12) adapted to vibrate the personal care device so that, in use, the personal care device vibrates with a vibration cycle having a vibration frequency;
a sensor arrangement (17) adapted to sense an operating parameter of the personal care device; and
a control unit (18) adapted to determine a target part of the vibration cycle based on the sensed operating parameter and to control the image capture device based on the target part of the vibration cycle.

2. The device of claim 1, wherein the personal care device comprises an oral care device, and wherein the image capture device (16) is adapted, in use, to capture images of one or more oral features of a user.

3. The device of claim 1 or 2, wherein the control unit (18) is adapted to control the image capture device to synchronise a timing of image capture with the target part of the vibration cycle.

4. The device of any preceding claim, wherein the control unit (18) is configured to generate a timestamp based on the target part of the vibration cycle, the timestamp being configured to identify a timing of the target part of the vibration cycle.

5. The device of any preceding claim, wherein the image capture device (16) comprises an illumination device (19) configured, in use, to illuminate a part of the user,
and preferably wherein the control unit (18) is adapted to control the illumination device to synchronise a timing of illumination with the target part of the vibration cycle.

6. The device of any preceding claim, wherein the image capture device (16) comprises a camera,
and wherein the control unit (18) is adapted to control the image capture device to synchronise signal acquisition from the camera with the target part of the vibration cycle.

7. The device of any preceding claim, wherein the vibratory means (12) comprises a vibrator,
wherein the sensor arrangement (17) comprises a drive sensor adapted to sense a drive signal of the vibrator,
and wherein the control unit (18) is adapted to determine the target part of the vibration cycle based on a time period when the sensed drive signal meets a predetermined drive signal requirement.

8. The device of claim 7, wherein the sensed drive signal is representative of a drive current or voltage of the vibrator, and wherein the predetermined requirement is that the magnitude of the drive current or voltage crosses a current or voltage threshold value.

9. The device of any of claims 1 to 7, wherein the sensor arrangement (17) comprises a movement sensor adapted to sense an angular velocity of a part of the personal care device,
and wherein the control unit is adapted to determine the target part of the vibration cycle based on a time period when the sensed angular velocity meets a predetermined velocity requirement, preferably wherein the velocity requirement is that the magnitude of the sensed angular velocity does not exceed a velocity threshold value.

10. The device of any of claims 1 to 7, wherein the sensor arrangement (17) comprises a hall sensor adapted to sense a magnetic field at a part of the personal care device,
and wherein the control unit is adapted to determine the target part of the vibration cycle based on a time period when then sensed magnetic field meets a predetermined magnetic field requirement.

11. The device of any of claims 1 to 10, wherein the sensor arrangement (17) comprises a vibration sensor adapted to sense an acceleration or excursion of a vibration of a part of the personal care device,
and wherein the control unit is adapted to determine the target part of the vibration cycle based on a time period when the acceleration or excursion of the sensed vibration meets a predetermined acceleration or excursion requirement.

12. The device of claim 11, wherein the predetermined requirement is that the acceleration or excursion of the sensed vibration exceeds a vibration threshold value.

13. The device of any of claims 1 to 12, wherein the personal care device comprises a toothbrush, a mouthpiece, a shaver or a skin cleansing brush.

14. A non-therapeutic method of controlling a personal care device, wherein the personal care device comprises: an image capture device adapted, in use, to capture images of one or more features of a user; vibratory means adapted to vibrate the personal care device so that, in use, the personal care device vibrates with a vibration cycle having a vibration frequency; and a sensor arrangement adapted to sense an operating parameter of the personal care device, and wherein the method comprises:
determining a target part of the vibration cycle based on the sensed operating parameter; and
controlling the image capture device based on the determined target part of the vibration cycle.

15. A computer program comprising computer program code means which is adapted, when said computer program is run on a personal care device, to implement the method claim 14.

## Patentansprüche

1. Körperpflegevorrichtung, umfassend:
eine Bildaufnahmevorrichtung (16), die dazu eingerichtet ist, in Verwendung Bilder von einem oder mehreren Merkmalen eines Benutzers aufzunehmen;
ein Vibrationsmittel (12), das dazu eingerichtet ist, die Körperpflegevorrichtung in Vibration zu versetzen, sodass die Körperpflegevorrichtung in Verwendung mit einem Vibrationszyklus vibriert, der eine Vibrationsfrequenz aufweist;
eine Sensoranordnung (17), die dazu eingerichtet ist, einen Betriebsparameter der Körperpflegevorrichtung zu erfassen; und
eine Steuereinheit (18), die dazu eingerichtet ist, einen Zielteil des Vibrationszyklus basierend auf dem erfassten Betriebsparameter zu bestimmen und die Bildaufnahmevorrichtung basierend auf dem Zielteil des Vibrationszyklus zu steuern.

2. Vorrichtung nach Anspruch 1, wobei die Körperpflegevorrichtung eine Mundpflegevorrichtung umfasst und wobei die Bildaufnahmevorrichtung (16) dazu eingerichtet ist, in Verwendung Bilder von einem oder mehreren Mundmerkmalen eines Benutzers zu erfassen.

3. Vorrichtung nach Anspruch 1 oder 2, wobei die Steuereinheit (18) dazu eingerichtet ist, die Bildaufnahmevorrichtung zu steuern, um eine Zeitsteuerung einer Bildaufnahme mit dem Zielteil des Vibrationszyklus zu synchronisieren.

4. Vorrichtung nach einem vorstehenden Anspruch, wobei die Steuereinheit (18) dazu konfiguriert ist, einen Zeitstempel basierend auf dem Zielteil des Vibrationszyklus zu erzeugen, wobei der Zeitstempel dazu konfiguriert ist, eine Zeitsteuerung des Zielteils des Vibrationszyklus zu identifizieren.

5. Vorrichtung nach einem vorstehenden Anspruch, wobei die Bildaufnahmevorrichtung (16) eine Beleuchtungsvorrichtung (19) umfasst, die dazu konfiguriert ist, in Verwendung einen Teil des Benutzers zu beleuchten,
und wobei vorzugsweise die Steuereinheit (18) dazu eingerichtet ist, die Beleuchtungsvorrichtung zu steuern, um eine Beleuchtungszeitsteuerung mit dem Zielteil des Vibrationszyklus zu synchronisieren.

6. Vorrichtung nach einem vorstehenden Anspruch, wobei die Bildaufnahmevorrichtung (16) eine Kamera umfasst,
und wobei die Steuereinheit (18) dazu eingerichtet ist, die Bildaufnahmevorrichtung zu steuern, um eine Signalerfassung von der Kamera mit dem Zielteil des Vibrationszyklus zu synchronisieren.

7. Vorrichtung nach einem vorstehenden Anspruch, wobei das Vibrationsmittel (12) einen Vibrator umfasst,
wobei die Sensoranordnung (17) einen Ansteuersensor umfasst, der dazu eingerichtet ist, ein Ansteuersignal des Vibrators zu erfassen,
und wobei die Steuereinheit (18) dazu eingerichtet ist, den Zielteil des Vibrationszyklus basierend auf einer Zeitperiode zu bestimmen, in der das erfasste Ansteuersignal eine vorbestimmte Ansteuersignalanforderung erfüllt.

8. Vorrichtung nach Anspruch 7, wobei das erfasste Ansteuersignal einen Ansteuerstrom oder eine Ansteuerspannung des Vibrators darstellt und wobei die vorbestimmte Anforderung ist, dass die Größe des Ansteuerstroms oder der Ansteuerspannung einen Strom- oder Spannungsschwellenwert kreuzt.

9. Vorrichtung nach einem der Ansprüche 1 bis 7, wobei die Sensoranordnung (17) einen Bewegungssensor umfasst, der dazu eingerichtet ist, eine Winkelgeschwindigkeit eines Teils der Körperpflegevorrichtung zu erfassen,
und wobei die Steuereinheit dazu eingerichtet ist, den Zielteil des Vibrationszyklus basierend auf einer Zeitperiode zu bestimmen, in der die erfasste Winkelgeschwindigkeit eine vorbestimmte Geschwindigkeitsanforderung erfüllt, wobei die Geschwindigkeitsanforderung vorzugsweise ist, dass die Größe der erfassten Winkelgeschwindigkeit einen Geschwindigkeitsschwellenwert nicht überschreitet.

10. Vorrichtung nach einem der Ansprüche 1 bis 7, wobei die Sensoranordnung (17) einen Hall-Sensor umfasst, der dazu eingerichtet ist, ein Magnetfeld an einem Teil der Körperpflegevorrichtung zu erfassen,
und wobei die Steuereinheit dazu eingerichtet ist, den Zielteil des Vibrationszyklus basierend auf einer Zeitperiode zu bestimmen, in der das erfasste Magnetfeld eine vorbestimmte Magnetfeldanforderung erfüllt.

11. Vorrichtung nach einem der Ansprüche 1 bis 10, wobei die Sensoranordnung (17) einen Vibrationssensor umfasst, der dazu eingerichtet ist, eine Beschleunigung oder Auslenkung einer Vibration eines Teils der Körperpflegevorrichtung zu erfassen,
und wobei die Steuereinheit dazu eingerichtet ist, den Zielteil des Vibrationszyklus basierend auf einer Zeitperiode zu bestimmen, in der die Beschleunigung oder Auslenkung der erfassten Vibration eine vorbestimmte Beschleunigungs- oder Auslenkungsanforderung erfüllt.

12. Vorrichtung nach Anspruch 11, wobei die vorbestimmte Anforderung ist, dass die Beschleunigung oder Auslenkung der erfassten Vibration einen Vibrationsschwellenwert überschreitet.

13. Vorrichtung nach einem der Ansprüche 1 bis 12, wobei die Körperpflegevorrichtung eine Zahnbürste, ein Mundstück, einen Rasierer oder eine Hautreinigungsbürste umfasst.

14. Nicht-therapeutisches Verfahren eines Steuerns einer Körperpflegevorrichtung, wobei die Körperpflegevorrichtung umfasst: eine Bildaufnahmevorrichtung, die dazu eingerichtet ist, in Verwendung Bilder von einem oder mehreren Merkmalen eines Benutzers aufzunehmen; ein Vibrationsmittel, das dazu eingerichtet ist, die Körperpflegevorrichtung in Vibration zu versetzen, sodass die Körperpflegevorrichtung in Verwendung mit einem Vibrationszyklus vibriert, der eine Vibrationsfrequenz aufweist; und eine Sensoranordnung, die dazu eingerichtet ist, einen Betriebsparameter der Körperpflegevorrichtung zu erfassen, und wobei das Verfahren umfasst:
Bestimmen eines Zielteils des Vibrationszyklus basierend auf dem erfassten Betriebsparameter; und
Steuern der Bildaufnahmevorrichtung basierend auf dem bestimmten Zielteil des Vibrationszyklus.

15. Computerprogramm, umfassend ein Computerprogrammcodemittel, das dazu eingerichtet ist, wenn das Computerprogramm auf einer Körperpflegevorrichtung läuft, den Verfahrensanspruch 14 zu implementieren.

## Revendications

1. Dispositif de soins personnels comprenant :
un dispositif de capture d'image (16) adapté, en cours d'utilisation, pour capturer des images d'une ou de plusieurs caractéristiques d'un utilisateur ;
des moyens vibratoires (12) adaptés pour faire vibrer le dispositif de soins personnels de sorte que, en cours d'utilisation, le dispositif de soins personnels vibre selon un cycle de vibration présentant une fréquence de vibration ;
un ensemble capteur (17) adapté pour détecter un paramètre de fonctionnement du dispositif de soins personnels ; et
une unité de commande (18) adaptée pour déterminer une partie cible du cycle de vibration sur la base du paramètre de fonctionnement détecté et pour commander le dispositif de capture d'image sur la base de la partie cible du cycle de vibration.

2. Dispositif selon la revendication 1, dans lequel le dispositif de soins personnels comprend un dispositif de soins buccaux, et dans lequel le dispositif de capture d'image (16) est adapté, en cours d'utilisation, pour capturer des images d'une ou plusieurs caractéristiques buccales d'un utilisateur.

3. Dispositif selon la revendication 1 ou 2, dans lequel l'unité de commande (18) est adaptée pour commander le dispositif de capture d'image afin de synchroniser un moment de la capture d'image avec la partie cible du cycle de vibration.

4. Dispositif selon une quelconque revendication précédente, dans lequel l'unité de commande (18) est configurée pour générer un horodatage sur la base de la partie cible du cycle de vibration, l'horodatage étant configuré pour identifier un moment de la partie cible du cycle de vibration.

5. Dispositif selon une quelconque revendication précédente, dans lequel le dispositif de capture d'image (16) comprend un dispositif d'éclairage (19) configuré, en cours d'utilisation, pour éclairer une partie de l'utilisateur,
et de préférence dans lequel l'unité de commande (18) est adaptée pour commander le dispositif d'éclairage afin de synchroniser un moment de l'éclairage avec la partie cible du cycle de vibration.

6. Dispositif selon une quelconque revendication précédente, dans lequel le dispositif de capture d'image (16) comprend une caméra,
et dans lequel l'unité de commande (18) est adaptée pour commander le dispositif de capture d'image afin de synchroniser l'acquisition du signal de la caméra avec la partie cible du cycle de vibration.

7. Dispositif selon une quelconque revendication précédente, dans lequel les moyens vibratoires (12) comprennent un vibrateur,
dans lequel l'ensemble capteur (17) comprend un capteur d'entraînement adapté pour détecter un signal d'entraînement du vibrateur,
et dans lequel l'unité de commande (18) est adaptée pour déterminer la partie cible du cycle de vibration sur la base d'une période de temps pendant laquelle le signal d'entraînement détecté répond à une exigence de signal d'entraînement prédéterminée.

8. Dispositif selon la revendication 7, dans lequel le signal d'entraînement détecté est représentatif d'un courant ou d'une tension d'entraînement du vibrateur, et dans lequel l'exigence prédéterminée est que l'ampleur du courant ou de la tension d'entraînement dépasse une valeur seuil de courant ou de tension.

9. Dispositif selon l'une quelconque des revendications 1 à 7, dans lequel l'ensemble capteur (17) comprend un capteur de mouvement adapté pour détecter une vitesse angulaire d'une partie du dispositif de soins personnels,
et dans lequel l'unité de commande est adaptée pour déterminer la partie cible du cycle de vibration sur la base d'une période de temps pendant laquelle la vitesse angulaire détectée répond à une exigence de vitesse prédéterminée, de préférence dans lequel l'exigence de vitesse est que l'ampleur de la vitesse angulaire détectée ne dépasse pas une valeur seuil de vitesse.

10. Dispositif selon l'une quelconque des revendications 1 à 7, dans lequel l'ensemble capteur (17) comprend un capteur à effet Hall adapté pour détecter un champ magnétique au niveau d'une partie du dispositif de soins personnels,
et dans lequel l'unité de commande est adaptée pour déterminer la partie cible du cycle de vibration sur la base d'une période de temps pendant laquelle le champ magnétique détecté répond à une exigence de champ magnétique prédéterminée.

11. Dispositif selon l'une quelconque des revendications 1 à 10, dans lequel l'ensemble capteur (17) comprend un capteur de vibrations adapté pour détecter une accélération ou une excursion d'une vibration d'une partie du dispositif de soins personnels,
et dans lequel l'unité de commande est adaptée pour déterminer la partie cible du cycle de vibration sur la base d'une période de temps pendant laquelle l'accélération ou l'excursion de la vibration détectée répond à une exigence d'accélération ou d'excursion prédéterminée.

12. Dispositif selon la revendication 11, dans lequel l'exigence prédéterminée est que l'accélération ou l'excursion de la vibration détectée dépasse une valeur seuil de vibration.

13. Dispositif selon l'une quelconque des revendications 1 à 12, dans lequel le dispositif de soins personnels comprend une brosse à dents, un embout buccal, un rasoir ou une brosse de nettoyage de la peau.

14. Procédé non thérapeutique de commande d'un dispositif de soins personnels, dans lequel le dispositif de soins personnels comprend : un dispositif de capture d'image adapté, en cours d'utilisation, pour capturer des images d'une ou plusieurs caractéristiques d'un utilisateur ; des moyens vibratoires adaptés pour faire vibrer le dispositif de soins personnels de sorte que, en cours d'utilisation, le dispositif de soins personnels vibre selon un cycle de vibration présentant une fréquence de vibration ; et un ensemble capteur adapté pour détecter un paramètre de fonctionnement du dispositif de soins personnels, et dans lequel le procédé comprend :
la détermination d'une partie cible du cycle de vibration sur la base du paramètre de fonctionnement détecté ; et
la commande du dispositif de capture d'image sur la base de la partie cible déterminée du cycle de vibration.

15. Programme informatique comprenant un moyen de code de programme informatique qui est adapté, lorsque ledit programme informatique est exécuté sur un dispositif de soins personnels, pour mettre en œuvre le procédé selon la revendication 14.
